(19) Europäisches Patentamt
European Patent Office
Office européen des brevets

(11) Veröffentlichungsnummer: **0 448 765 B1**

# (12) EUROPÄISCHE PATENTSCHRIFT

(45) Veröffentlichungstag der Patentschrift: **17.08.94**

(51) Int. Cl.5: **A61K 31/415**, A61K 31/44

(21) Anmeldenummer: **90106200.0**

(22) Anmeldetag: **30.03.90**

## (54) Verwendung von Guanidinderivaten zur Herstellung eines Arzneimittels mit NPY-antagonistischer Wirkung.

(43) Veröffentlichungstag der Anmeldung:
**02.10.91 Patentblatt 91/40**

(45) Bekanntmachung des Hinweises auf die Patenterteilung:
**17.08.94 Patentblatt 94/33**

(84) Benannte Vertragsstaaten:
**AT BE CH DE DK ES FR GB GR IT LI LU NL SE**

(56) Entgegenhaltungen:
**EP-A- 0 199 845**
**EP-A- 0 262 448**

**Arch. Pharm. (Weinheim), Band 321, 1988,Seiten 415-418, VCH Verlagsgesellschaft mbH, Weinheim, DE; A. Buschauer: "Synthese positiv inotroper subs- tanzen: Imidazolylpropylguanadine mit pyridinpartialstruktur"**

**J. Med. Chem. Band 32, 1989, Seiten 1963-1970, American Chemical Society; A. Buschauer: "Synthesis and in vitro pharmacology of arpromidine and related phenyl (pyridylalkyl) guanidines, a ptential new class of positive inotropicdrugs"**

(73) Patentinhaber: **HEUMANN PHARMA GMBH & CO**
**Heideloffstrasse 18 - 28**
**D-90478 Nürnberg (DE)**

(72) Erfinder: **Michel, Martin C., Dr.**
**Bochumer Strasse 10**
**W-4300 Essen 14 (DE)**
Erfinder: **Mörsdorf, Johann Peter, Dr., Dipl.-Chem.**
**Sportplatzstrasse 4**
**W-8506 Langenzenn (DE)**
Erfinder: **Engler, Heidrun, Dr.**
**1266 Whispering Pines**
**St. Louis, MO 6346 (DE)**
Erfinder: **Schickaneder, Helmut, Dr., Dipl.-Chem.**
**Wiesenstrasse 16**
**W-8501 Eckental (DE)**
Erfinder: **Ahrens, Kurt-Henning, Dr.**
**Trödelmarkt 42**
**D-8500 Nürnberg (DE)**

(74) Vertreter: **Kraus, Walter, Dr. et al**
**Patentanwälte Kraus, Weisert & Partner**
**Thomas-Wimmer-Ring 15**
**D-80539 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Zofia Zukowska-Grojec, Eric S. Marks, Markus Haass, Am. J. Physiol. Nov. 1987, pp. H1234-1239**

## Beschreibung

Neuropeptid Y (NPY) ist ein Peptid aus 36 Aminosäuren, das ursprünglich aus Schweinehirnen isoliert wurde (Tatemoto K., Proc. Natl. Sci., USA 79, 5485 (1982)), aber auch beim Menschen im zentralen und peripheren Nervensystem gefunden wurde.

Zusammen mit Noradrenalin steuert NPY den vaskulären sympathischen Tonus. Die systemische Anwendung vom NPY führt zu einem langanhaltenden Anstieg des Gefäßwiderstandes. Von Boublik et al. (Boublik, J.H., Scott, N.A., Brown, M.R. und Rivier, J.E., J. Med. Chem. 32, 597 (1989)) wurde auch die Beteiligung von NPY bei der Entstehung des Bluthochdruckes nachgewiesen. Aus Zukowska-Grojec et al. (Am. J. Physiol. Nov 1987, H 1234-1239) ist bekannt daß NPY bei der Entstehung von myocardischer Ischämie sowie der sogenannten "heart failure" beteiligt ist.

NPY-Antagonisten stellen daher einen potentiellen neuen Weg in der Behandlung des Bluthochdruckes dar. Bisher waren jedoch keine NPY-Antagonisten bekannt.

Aus den DE-OS'en 35 12 084, 35 28 214, 35 28 215 und 36 31 334 sowie der EP-A-0 199 845 sind bereits Guanidinderivate mit folgender Grundstruktur

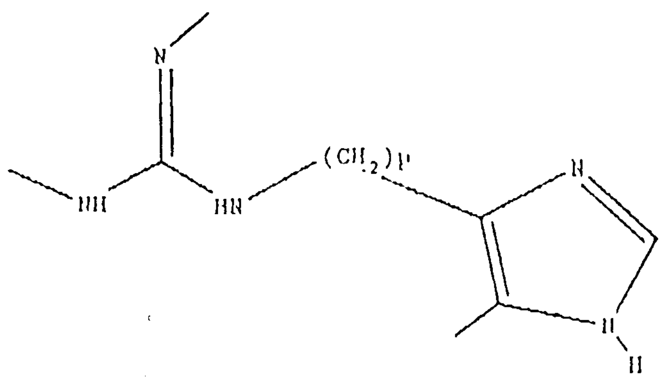

(I)

bekannt, welche Histamin-$H_2$-agonistische und Histamin-$H_1$-antagonistische Wirkungen haben. Nach den Angaben dieser Druckschriften eignen sich diese Verbindungen aufgrund ihrer pharmakologischen Eigenschaften als Cardiotonika, d.h. die Kontraktionskraft des Herzens steigernde Verbindungen. Demgemäß werden sie für die Therapie der akuten und der chronischen Herzinsuffizienz vorgeschlagen.

Es wurde nun gefunden, daß überraschenderweise die vorstehend beschriebenen Verbindungen unabhängig von der oben aufgeführten cardiotonischen und positiv inotropen Wirkung auch Neuropeptid-Y-antagonistische Wirkungen aufweisen.

Gegenstand der vorliegenden Erfindung ist daher die Verwendung von Guanidinderivaten der allgemeinen Formel II

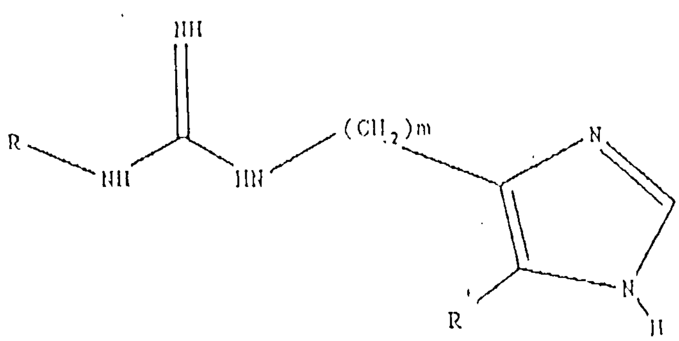

(II)

in der R die Gruppierung

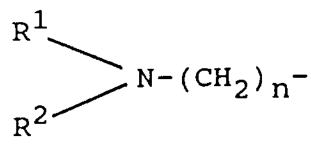

bedeutet, wobei $R^1$ für eine unsubstituierte oder eine ein- oder zweifach mit Halogenatomen, $C_1$-$C_3$-Alkylgruppen oder $C_1$-$C_3$-Alkoxygruppen substituierte Phenylgruppe oder einen unsubstituierten oder einen ein- oder zweifach mit Halogenatomen, $C_1$-$C_3$-Alkylgruppen oder $C_1$-$C_3$-Alkoxygruppen substituierten Pyridinring steht, $R^2$ für ein Wasserstoffatom, eine $C_1$-$C_3$-Alkylgruppe, eine gegebenenfalls ein- oder zweifach mit Halogenatomen, $C_1$-$C_3$-Alkylgruppen, $C_1$-$C_3$-Alkoxygruppen substituierte Phenylgruppe oder eine unsubstituierte oder ein- oder zweifach mit Halogenatomen, $C_1$-$C_3$-Alkylgruppen oder $C_1$-$C_3$-Alkoxygruppen substituierte Benzyl- oder Heteroarylmethylgruppe steht und n den Wert 2, 3 oder 4 hat, oder
in der R die Gruppierung

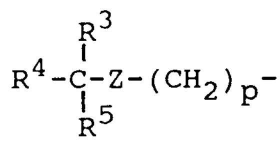

bedeutet, worin $R^3$ für einen unsubstituierten oder ein- oder zweifach mit Halogenatomen, $C_1$-$C_3$-Alkylgruppen oder $C_1$-$C_3$-Alkoxygruppen substituierten Phenylring oder Pyridinring steht, $R^4$ ein Wasserstoffatom oder eine gegebenenfalls ein- oder zweifach mit Halogenatomen, $C_1$-$C_3$-Alkylgruppen oder $C_1$-$C_3$-Alkoxygruppen substituierte Phenylgruppe bedeutet, $R^5$ für ein Wasserstoffatom oder eine Methyl- oder Hydroxygruppe und Z für eine Einfachbindung, ein Sauerstoffatom oder ein Schwefelatom stehen und p den Wert 2 oder 3 hat,
m den Wert 2 oder 3 hat und R' ein Wasserstoffatom oder eine Methylgruppe bedeutet,
sowie der physiologisch annehmbaren Salze davon zur Herstellung eines Arzneimittels mit NPY-antagonistischer Wirkung.

Die Neuropeptid-Y-antagonistische Wirkung der erffindungsgemäß in Betracht gezogenen Substanzen stellt eine Erklärung dafür dar, warum diese insbesondere zur Behandlung des Bluthochdrucks geeignet sind. Siehe EP-A-0 262 448 und EP-A-0 199 845.

In der oben angegebenen allgemeinen Formel II kann R die Gruppierung

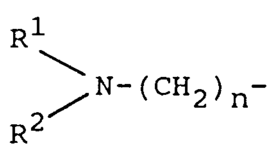

bedeuten. In dieser Gruppierung steht $R^1$ für eine unsubstituierte oder eine ein- oder zweifach substituierte Phenylgruppe. Im Falle der Substitution kommen insbesondere 1 bis 2 Halogenatome, wie Fluor-, Chlor- oder Bromatome, vorzugsweise Fluor- oder Chloratome, 1 bis 2 $C_1$-$C_3$-Alkylgruppen, vorzugsweise Methyl- oder Ethylgruppen, und 1 bis 2 $C_1$-$C_3$-Alkoxygruppen, wie Methoxy- oder Ethoxygruppen, in Betracht. Im Falle der Einfachsubstitution wird die Substitution in 4-Position und im Falle der Zweifachsubstitution die Substitution in 3- oder 4-Position bzw. 3- und 5-Position des Phenylrings bevorzugt.

Der Substituent $R^1$ kann weiterhin ein unsubstituierter oder ein ein- bis zweifach substituierter Pyridinring sein. Als Substituent des Pyridinrings kommen beispielsweise Halogenatome, wie Fluor-, Chlor- oder Bromatome, vorzugsweise Brom- oder Chloratome, ganz besonders bevorzugt Bromatome, $C_1$-$C_3$-Alkylgruppen, wie Methyl- oder Ethylgruppen, sowie $C_1$-$C_3$-Alkoxygruppen, wie Methoxy-, Ethoxy- oder Propoxygruppen, vorzugsweise Methoxygruppen, in Betracht.

Die Verknüpfung des durch $R^1$ angegebenen Pyridinrings mit dem Stickstoffatom in der Gruppierung R kann in 2-, 3- oder 4-Position des Pyridinrings erfolgen, wobei die 2- oder 3-Position bevorzugt wird. Dabei wird die Verknüpfung in der 2-Position des Pyridinrings ganz besonders bevorzugt.

$R^2$ steht für ein Wasserstoffatom, eine $C_1$-$C_3$-Alkylgruppe, insbesondere eine Methyl-, Ethyl- oder Propylgruppe, eine Phenylgruppe, die unsubstituiert sein kann oder gegebenenfalls ein- oder zweifach substituiert, eine Benzylgruppe, die unsubstituiert oder ein- oder zweifach substituiert sein kann, oder eine Heteroarylmethylgruppe, die unsubstituiert sein kann oder ein- oder zweifach substituiert sein kann. Im Falle der Substitution kann die durch $R^2$ angegebene Phenylgruppe in der gleichen Weise und mit den gleichen Substituenten wie oben im Zusammenhang mit der Substitution der durch $R^1$ angegebenen Phenylgruppe beschrieben substituiert sein.

Im Falle der Substitution kann die Benzylgruppe mit 1 bis 2 Halogenatomen, wie Fluor-, Chlor- oder Bromatomen, vorzugsweise Chlor- oder Fluoratomen, oder $C_1$-$C_3$-Alkoxygruppen, wie Methoxy- oder Ethoxygruppen, vorzugsweise Methoxygruppen, substituiert sein. Im Falle der Einfachsubstitution der durch $R^2$ angegebenen Benzylgruppe ist der Substituent vorzugsweise in para-Position zur Methylengruppe gebunden, während im Falle der Zweifachsubstitution die 3- und 4-Position der Benzylgruppe bevorzugt wird.

Wenn $R^2$ eine Heteroarylmethylgruppe darstellt, dann ist diese Gruppe vorzugsweise eine Thiophenylmethyl-, Furanmethyl- oder Pyridinmethylgruppe. Auch die Heteroarylmethylgruppe kann unsubstituiert oder vorzugsweise ein- oder zweifach substituiert sein. Als Substituenten kommen Halogenatome, wie Fluor-, Chlor- oder Bromatome, $C_1$-$C_3$-Alkylgruppen, wie Methyl- oder Ethylgruppen, und lineare $C_1$-$C_3$-Alkoxygruppen, wie Methoxygruppen, in Betracht.

Der Index n hat den Wert 2, 3 oder 4, wobei der Wert 3 bevorzugt wird.

R kann weiterhin die Gruppierung

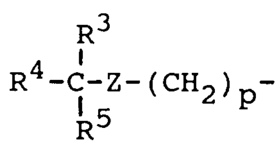

bedeuten. In dieser Gruppierung kann $R^3$ eine unsubstituierte oder eine ein- oder zweifach substituierte Phenylgruppe oder einen unsubstituierten oder ein- oder zweifach substituierten Pyridinring bedeuten. Im Falle der Substitution kommen insbesondere 1 bis 2 Halogenatome, wie Fluor-, Chlor- oder Bromatome, vorzugsweise Fluor- oder Chloratome, 1 bis 2 $C_1$-$C_3$-Alkylgruppen, vorzugsweise Methyl- oder Ethylgruppen, und 1 bis 2 $C_1$-$C_3$-Alkoxygruppen, wie Methoxy- oder Ethoxygruppen, in Betracht. Die Einfachsubstitution und die Zweifachsubstitution werden bevorzugt. Im Falle der Einfachsubstitution wird die Substitution in 4-Position und im Falle der Zweifachsubstitution wird die Substitution in 3- und 4-Position des Phenylrings bevorzugt. Der Substituent $R^3$ kann weiterhin ein unsubstituierter oder ein ein- oder zweifach substituierter Pyridinring, vorzugsweise ein unsubstituierter Pyridinring oder ein einfach substituierter Pyridinring sein. Als Substituenten des Pyridinrings kommen beispielsweise Halogenatome, wie Fluor-, Chlor- oder Bromatome, vorzugsweise Brom- oder Chloratome, ganz besonders bevorzugt Bromatome, $C_1$-$C_3$-Alkylgruppen, wie Methyl- oder Ethylgruppen, sowie $C_1$-$C_3$-Alkoxygruppen, wie Methoxy-, Ethoxy- oder Propoxygruppen, vorzugsweise Methoxygruppen, in Betracht.

Die Verknüpfung des durch $R^3$ angegebenen Pyridinrings mit dem Kohlenstoffatom in der Gruppierung R kann in 2-, 3- oder 4-Position des Pyridinrings erfolgen, wobei die 2- oder 3-Position bevorzugt wird. Dabei wird die Verknüpfung in der 2-Position des Pyridinrings ganz besonders bevorzugt.

$R^4$ bedeutet ein Wasserstoffatom oder eine unsubstituierte oder eine ein- oder zweifach substituierte Phenylgruppe. Im Falle der Substitution ist die durch $R^4$ angegebene Phenylgruppe in der gleichen Weise substituiert wie die durch $R^3$ angegebene Phenylgruppe. $R^5$ bedeutet ein Wasserstoffatom oder eine Methyl- oder Hydroxygruppe. Z stellt eine Einfachbindung, ein Sauerstoffatom oder ein Schwefelatom dar, während p den Wert 2 oder 3 hat.

In der allgemeinen Formel II hat m den Wert 2 oder 3, vorzugsweise 3, und R' bedeutet ein Wasserstoffatom oder eine Methylgruppe, vorzugsweise ein Wasserstoffatom.

Erfindungsgemäß wird die Verwendung von Guanidinderivaten der oben angegebenen allgemeinen Formel II bevorzugt, bei denen R für eine der Gruppen
2-(Diphenylmethoxy)ethyl, 2-[Bis-(4-fluorphenyl)-methoxy]ethyl,
2-[Bis-(4-chlorphenyl)methoxy]ethyl,
3-(4-Fluorphenyl)-3-(pyridin-2-yl)propyl,

3-(3,4-Difluorphenyl)-3-(pyridin-2-yl)propyl,
3-(3,5-Difluorphenyl)-3-(pyridin-2-yl)propyl,
3-(4-Chlorphenyl)-3-(pyridin-2-yl)propyl,
3-(3,4-Dichlorphenyl)-3-(pyridin-2-yl)propyl,
3-(4-Fluorphenyl)-3-(pyridin-3-yl)propyl,
2-[N-(5-Brom-3-methyl-pyridin-2-yl)-benzylamino]ethyl
2-[N-(5-Brom-3-methyl-pyridin-2-yl)-(4-chlorbenzyl)amino]ethyl
4-(5-Brom-3-methyl-pyridin-2-yl)butyl,
3-(5-Brom-3-methyl-pyridin-2-yl)propyl,
4-(5-Brom-pyridin-2-yl)butyl,
3-(5-Brom-pyridin-2-yl)propyl,
3-(4-Chlorphenyl)-3-phenylpropyl,
3-(4-Fluorphenyl)-3-phenylpropyl,
3,3-Bis-(4-fluorphenyl)propyl oder
3,3-Bis-(4-chlorphenyl)propyl
steht.

Ganz besonders wird die Verwendung der nachstehend angegebenen Einzelverbindungen bevorzugt:

$N^1$-[3-(1H-Imidazol-4-yl)propyl]-$N^2$-[2-[(pyridin-3-yl)methylthio]ethyl]-guanidin
$N^1$-[3-(1H-Imidazol-4-yl)propyl]-$N^2$-(3,3-diphenylpropyl)-guanidin
$N^1$-[3-(1H-Imidazol-4-yl)]propyl]-$N^2$-[2-[(pyridin-2-yl)-amino]ethyl]-guanidin
$N^1$-[3-[(5-Brom-3-methyl-pyridin-2-yl)amino]propyl]-$N^2$-[3-(1H-imidazol-4-yl)propyl]-guanidin
$N^1$-[3-(1H-Imidazol-4-yl)propyl]-$N^2$-[2-(diphenylmethoxy)ethyl]-guanidin (Verbindung A)
$N^1$-[3-(3,5-Difluorphenyl)-3-(pyridin-2-yl)propyl]-$N^2$-[3-(1H-imidazol-4-yl)propyl]-guanidin (Verbindung B)
$N^1$-[2-[N-(5-Brom-3-methyl-pyridin-2-yl)-benzylamino]ethyl]-$N^2$-[3-(1H-imidazol-4-yl)propyl]-guanidin (Verbindung C)
$N^1$-[4-(5-Brom-3-methyl-pyridin-2-yl)butyl]-$N^2$-[3(1H-imidazol-4-yl)propyl]-guanidin (Verbindung D)

Bei den erfindungsgemäß verwendeten Verbindungen handelt es sich um bekannte Verbindungen, die nach den in den oben angegebenen Druckschriften beschriebenen Verfahren herstellbar sind.

Die Neuropeptid-Y-antagonistische Wirkung der erfindungsgemäß verwendeten Verbindungen wurde anhand der Methode von Motulsky und Michel (Motulsky, H.J., Michel, M.C., Am. J. Physiol. 255, 880 (1988)) nachgewiesen.

Bei dieser Methode wird der durch NPY induzierte Anstieg der intrazellulären $Ca^{++}$-Konzentration in HEL-Zellen (Human Erythroleukemia-Zellen) fluorimetrisch mit Fura-2 als Indikator gemessen. Unter den gegebenen Bedingungen bewirkt NPY einen konzentrationsabhängigen Anstieg der intrazellulären $Ca^{++}$-Konzentration über die Stimulation des spezifischen NPY-Rezeptors.

Zur Messung der Hemmwirkung der zu testenden Antagonisten werden diese in Konzentrationen von $10^{-4}$ bis $10^{-6}$ dem Inkubationsmedium zugesetzt. Dann wird erneut eine NPY-Wirkungskurve bestimmt.

Die erfindungsgemäß verwendeten Guanidinverbindungen verschieben die NPY-Konzentrationswirkungskurve nach rechts. Die Rechtsverschiebung ist nach Schild-Plot-Analyse kompetitiv. Die Substanzen antagonisieren demnach die NPY-Wirkung durch Konkurrenz am spezifischen NPY-Rezeptor.

Die folgende Tabelle gibt die Meßwerte als $pA_2$-Werte wieder:

| Verbindung | $pA_2$ Hemmung des $Ca^{++}$-Anstieges |
|---|---|
| A | 4,0 |
| B | 4,72 |
| C | 5,88 |
| D | 5,04 |

Bei dem oben beschriebenen Test, deren Ergebnisse in der Tabelle dargestellt sind, wurden die folgenden Verbindungen verwendet:

Verbindung A: $N^1$-[3-(1H-Imidazol-4-yl)propyl]-$N^2$-[2-(diphenylmethoxy)ethyl]-guanidin

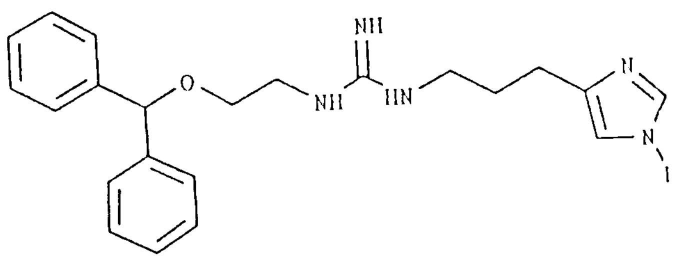

Verbindung B: $N^1$-[3-(3,5-Difluorphenyl)-3-(pyridin-2-yl)propyl]-$N^2$-[3-(1H-imidazol-4-yl)propyl]-guanidin

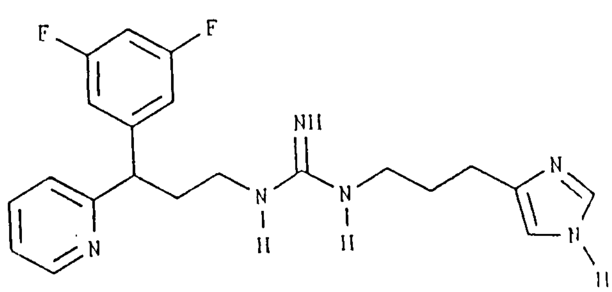

Verbindung C: $N^1$-[2-[N-(5-Brom-3-methyl-pyridin-2-yl)benzylamino]-ethyl]-$N^2$-[3-(1H-imidazol-4-yl)propyl]-guanidin

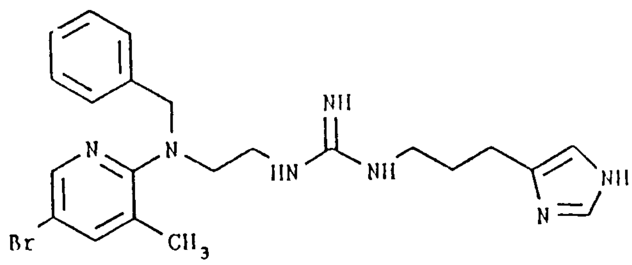

Verbindung D: $N^1$-[4-(5-Brom-3-methyl-pyridin-2-yl)butyl]-$N^2$-[3-(1H-imidazol-4-yl)propyl]-guanidin

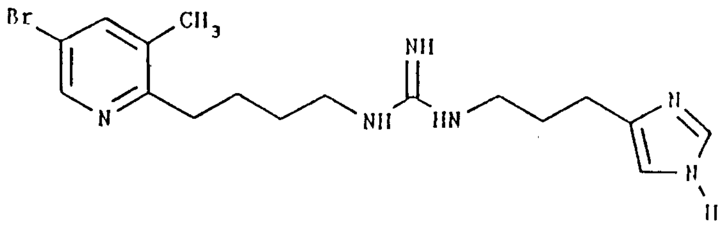

Die Erfindung wird in den Beispielen beschrieben.

Beispiel 1

$N^1$-[3-(1H-Imidazol-4-yl)propyl]-$N^2$-[2-[(pyridin-3-yl)methylthio]ethyl]guanidin-trihydrochlorid

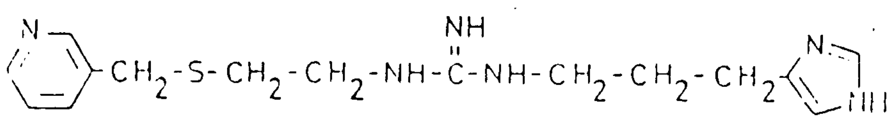 x 3 HCl

0,85 g (2 mmol) N-Benzoyl-N'-[3-(imidazol-4-yl)propyl]-N''-{2-[(pyrid-3-yl)methylthio]ethyl}guanidin werden 6 Stunden in 45 ml 18%iger Salzsäure unter Rückfluß erhitzt. Nach dem Erkalten des Reaktionsansatzes wird die entstandene Benzoesäure durch Extraktion mit Ether entfernt, die wäßrige Phase im Vakuum zur Trockene eingedampft und der Rückstand im Hochvakuum getrocknet. Man erhält 0,78 g (91%) trockenen, stark hygroskopischen Schaum.

$C_{15}H_{22}N_6S \cdot 3HCl$ (427,8)

Molmasse (MS): ber.: 318,16267; gef.: 318,16299

MS: m/z (rel. Int. [%]) = 318($M^+$, 3), 168(17), 125(29), 95(51), 93(100), 92(57), 44(89).

| | |
|---|---|
| $^1$H-NMR-Daten (d$_6$-DMSO, TMS als interner Standard) | δ = 1,87 (m) 2 H,<br>2,62 (t) 2 H,<br>2,73 (t) 2 H,<br>3,0 - 3,7 (m) 4 H,<br>4,10 (s) 2 H,<br>7,3 - 8,3 (m) 6 H, 4 H austauschbar mit $D_2O$<br>8,5 - 9,1 (m) 4 H, ppm. |

Beispiel 2

N[1]-[3-(1H-Imidazol-4-yl)propyl]-N[2]-(3,3-diphenylpropyl)guanidin-dihydrochlorid

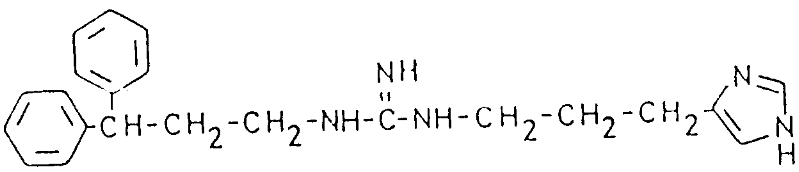

x 2 HCl

0,84 g (1,8 mmol) N-Benzoyl-N'-[3-(imidazol-4-yl)propyl]-N''-(3,3-diphenylpropyl)guanidin werden in 45 ml 20%iger Salzsäure 7 Stunden unter Rückfluß erhitzt. Die Aufarbeitung erfolgt wie in Beispiel 1.
Ausb.: 0,67 g (86%) hygroskopischer, nichtkristalliner Feststoff.
$C_{22}H_{27}N_5 \cdot 2HCl$ (434,4)
MS: m/z (rel. Int. [%]) = 362([M+H]$^+$, 84), 167(54), 109(100), 91(60) (FAB-Methode).

| | |
|---|---|
| $^1$H-NMR-Daten: (d$_6$-DMSO, TMS als interner Standard) | δ = 1,81 (m) 2 H,<br>2,27 (dt) 2 H,<br>2,68 (t) 2 H,<br>3,02 (m) 2 H,<br>3,16 (m) 2 H,<br>4,10 (t) 1 H,<br>7,15 - 7,6 (m) 13 H,<br>2 H, austauschbar mit $D_2O$,<br>7,80 (m) 2 H, austauschbar mit $D_2O$,<br>8,99 (d) 1 H, ppm. |

Beispiel 3

N[1][3-(1H-Imidazol-4-yl)propyl]-N[2]-[2-(pyridin-2-yl-amino)ethyl]-guanidin-trihydrochlorid

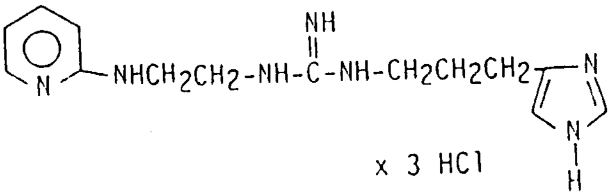

Aus 1,21 g (3,1 mmol) N[1]-Benzoyl-N[2]-[3-(1H-imidazol-4-yl)propyl]-N[3]-[2-(pyridin-2-yl-amino)ethyl]-guanidin und 20 ml konz. Salzsäure werden 0.93 g (76%) eines farblosen, hygroskopischen Feststoffes erhalten.
$C_{14}H_{24}Cl_3N_7$ (396,75)

| | |
|---|---|
| $^1$H-NMR-Daten: (CD$_3$OD, TMS als interner Standard) | δ = 1,80 - 2,21 (m) 2 H,<br>2,69 - 3,00 (m) 2 H,<br>3,37 (t) 2 H,<br>3,57 - 3,83 (m) 4 H,<br>4,8 (breit) 8 H, austauschbar mit $D_2O$,<br>6,96 (t) 1 H,<br>7,22 (d) 1 H,<br>7,44 (s) 1 H,<br>7,83 - 8,16 (m) 2 H,<br>8,87 (s) 1 H, ppm. |

Beispiel 4

$N^1$-[3-[(5-Brom-3-methyl-pyridin-2-yl)amino]propyl]-$N^2$-[3-(1H-imidazol-4-yl)propyl]-guanidin-hydroiodid

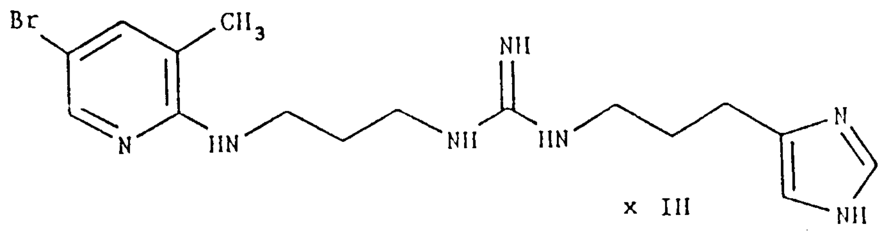

1,50 g (3,37 mmol) 3-[(5-Brom-3-methyl-pyridin-2-yl)amino)propyl]-isothiuroniumiodid und 0,42 g (3,37 mmol) 3-(1H-Imidazol-4-yl)propylamin werden in 20 ml Acetonitril 3 Stunden unter Rückfluß gekocht.

Nach Abkühlen wird das Reaktionsgemisch im Vakuum eingedampft und der Rückstand an Kieselgel mit Ethylacetat/Methanol (70:30) als Laufmittel chromatographisch gereinigt. Die Hauptfraktion ergibt nach Eindampfen im Vakuum 0,41 g (23%) eines farblosen, amorphen Feststoffes.
$C_{16}H_{25}BrJN_7$ (522,24)

$^1$H-NMR-Daten: (CD$_3$OD, TMS als interner Standard)

δ = 1,93 (m) 4 H
2,12 (s) 3 H
2,69 (t) 2 H
3,2 - 3,6 (m) 6 H
4,9 (breit) 6 H,

austauschbar mit $D_2O$
6,95 (s) 1 H
7,40 (d) 1 H
7,69 (s) 1 H
7,93 (d) 1 H, ppm.

Beispiel 5

$N^1$-[3-(1H-Imidazol-4-yl)propyl]-$N^2$-[2-(diphenylmethoxy)ethyl]-guanidin-hydroiodid

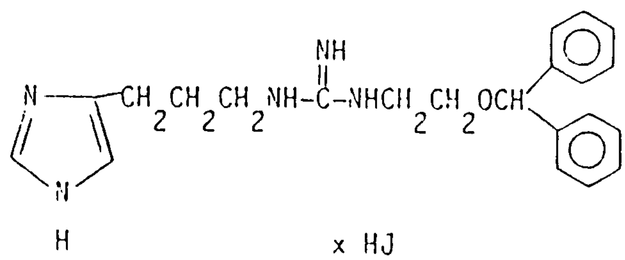

x HJ

a) $N^1$-Benzoyl-$N^2$-[2-(diphenylmethoxy)ethyl]-thioharnstoff

7,8 g (34 mmol) 2-(Diphenylmethoxy)-ethylamin und 5,6 g (34 mmol) Benzoylisothiocyanat werden in 60 ml Ethylacetat 2 Stunden bei Raumtemperatur gerührt. Der ausgefallene Feststoff wird abgesaugt, mit etwas Ethylacetat gewaschen und aus Ethanol umkristallisiert. Man erhält 11,1 g (83%) farblose Kristalle, FP. 126 - 127 °C.
$C_{23}H_{22}N_2O_2S$ (390,5)

b) S-Methyl-N-[2-(diphenylmethoxy)ethyl]-isothiuroniumiodid

11,1 g (28 mmol) $N^1$-Benzoyl-$N^2$-[2-(diphenylmethoxy)ethyl]-thioharnstoffwerden in 200 ml Methanol und 60 ml Wasser mit 4,15 g (30 mmol) Kaliumcarbonat 40 Minuten gekocht. Nach Abdampfen der Lösungsmittel im Vakuum wird der Rückstand in 20 ml Wasser aufgenommen und die wäßrige Phase viermal mit 30 ml Dichlormethan extrahiert. Die vereinigten organischen Phasen werden mit Natriumsulfat getrocknet, filtriert und im Vakuum eingedampft. Der Rückstand wird in 100 ml Ethanol aufgenommen und mit 2,1 ml (33 mmol) Methyliodid 20 Stunden bei Raumtemperatur gerührt. Nach Abdampfen des Lösungsmittels im Vakuum erhält man 11,4 g (94%) eines farblosen, hochviskosen Öls.
$C_{17}H_{21}JN_2OS$ (428,3)

c) $N^1$-[3-(1H-Imidazol-4-yl)propyl]-$N^2$-[2-(diphenylmethoxy)ethyl]-guanidin-hydroiodid

1,73 g (4 mmol) S-Methyl-N-[2-(diphenylmethoxy)ethyl]-isothiuroniumiodid und 0,50 g (4 mmol) 3-(1H-Imidazol-4-yl)propylamin werden in 20 ml Acetonitril 3 Stunden unter Rückfluß gekocht. Nach Abdampfen des Lösungsmittels im Vakuum und nach chromatographischer Reinigung an Kieselgel mit Dichlormethan/Methanol (80:20) als Laufmittel werden 1,41 g (70%) eines farblosen amorphen Feststoffs erhalten.
$C_{22}H_{28}JN_5O$ (505,4)

| | |
|---|---|
| $^1$H-NMR-Daten: (CD$_3$OD, TMS als interner Standard) | $\delta$ = 1,7 - 2,1 (m) 2 H,<br>2,7 (t) 2 H,<br>3,1 - 3,8 (m) 6 H,<br>4,9 (breit) 5 H, austauschbar mit $D_2O$,<br>5,6 (s) 1 H,<br>7,0 (s) 1 H,<br>7,2 - 7,6 (m) 10 H<br>8,0 (s) 1 H, ppm. |

Beispiel 6

N[1]-[3-(3,5-Difluorphenyl)-3-(pyridin-2-yl)propyl]-N[2]-[3-(1H-imidazol-4-yl)propyl]guanidin-trihydrochlorid

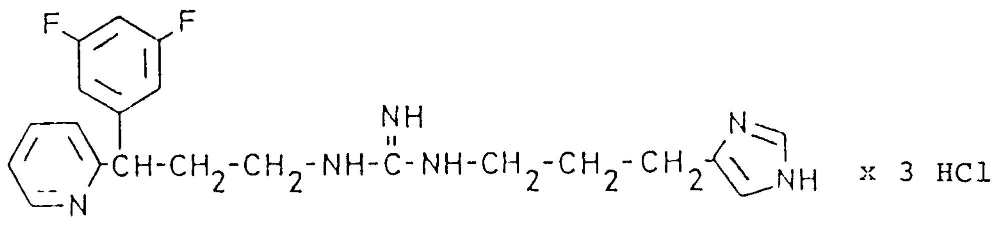

a) N[1]-Benzoyl-N[2]-[3-(3,5-difluorphenyl)-3-(pyridin-2-yl) propyl]-N[3]-[3-(1H-imidazol-4-yl) propyl]guanidin

1,24 g 3-(3,5-Difluorphenyl)-3-(pyridin-2-yl)propylamin und 1,59 g (5 mmol) N-Benzoyl-diphenylimidocarbonat werden in 20 ml Methylenchlorid 20 Minuten bei Raumtemperatur gerührt. Anschließend wird das Lösungsmittel im Vakuum abdestilliert, der ölige Rückstand in 30 ml Pyridin aufgenommen und nach Zusatz von 0,65 g (5,2 mmol) 3-(1H-Imidazol-4-yl)propylamin 45 Minuten auf 100 °C erhitzt. Der Reaktionsansatz wird im Vakuum eingedampft, der Rückstand in 5%iger Salzsäure aufgenommen und mit Ether extrahiert. Anschließend wird mit Ammoniak alkalisiert, mit Methylenchlorid ausgeschüttelt, die organische Phase mit Wasser gewaschen, über Natriumsulfat getrocknet und im Vakuum eingedampft. Das Reaktionsprodukt wird durch präparative Schichtchromatographie an Kieselgel 60 $PF_{254}$ gipshaltig isoliert und gereinigt. (Fließmittel: Chloroform/Methanol, 99,5:0,5, Ammoniakatmosphäre). Nach Eindampfen der Eluate werden 1,3 g (52%) eines farblosen amorphen Feststoffs erhalten.
$C_{28}H_{28}F_2N_6O$ (502,6)

$^1$H-NMR-Daten: ($CDCl_3$, TMS als interner Standard)

$\delta$ = 1,96 (m) 2 H,
2,3 (breit) 1 H,
2,6 - 2,8 (m) 3 H,
3,34 (breit ) 2 H,
3,5 (breit) 1 H,
3,9 (breit) 1 H,
4,17 (dd) 1 H,
6,6 - 7,8 (m) 11 H,
8,12 (d) 2 H,
8,58 (d) 1 H,
10,3 - 10,9 (breit) 1 H, austauschbar mit $D_2O$, ppm.

b) $N^1$-[3-(3,5-Difluorphenyl)-3-(pyridin-2-yl)propyl]-$N^2$-[3-(1H-imidazol-4-yl)propyl]guanidin

0,76 g (1,5 mmol) $N^1$-Benzoyl-$N^2$-[3-(3,5-difluorphenyl)-3-(pyridin-2-yl)propyl]-$N^3$-[3-(1H-imidazol-4-yl)-propyl]guanidin werden in 40 ml 20%iger Salzsäure 10 Stunden unter Rückfluß erhitzt. Anschließend wird die salzsaure Lösung dreimal mit Ether extrahiert, im Vakuum zur Trockene eingedampft und im Hochvakuum getrocknet.
Ausbeute: 0,65 g (85%) des Trihydrochlorids in Form eines hygroskopischen amorphen Feststoffes.
$C_{21}H_{24}F_2N_6 \cdot 3HCl$ (507,8)
MS (FAB-Methode): m/z (rel. Int. [%]) = 399 ([M + H]$^+$,80), 232 (100), 204 (18), 109 (60), 100 (36), 95 (11).

| | |
|---|---|
| $^1$H-NMR-Daten: (DMSO-$d_6$, TMS als interner Standard) | δ = 1,85 (m) 2 H,<br>2,35 - 2,65 (m) 2 H,<br>2,72 (t) 2 H,<br>3,0 - 3,3 (m) 4 H,<br>4,78 (t) 1 H,<br>7,16 (dd) 1 H,<br>7,36 (d) 2 H,<br>7,51 (s) 1 H,<br>7,62 (s) 2 H, austauschbar mit $D_2O$,<br>7,76 (dd) 1 H,<br>8,02 (m) 3 H, 2 H, austauschbar mit $D_2O$,<br>8,32 (dd) 1 H,<br>8,75 (d) 1 H,<br>9,05 (s) 1 H,<br>14,45 (breit) 1 H, austauschbar mit $D_2O$,<br>14,8 (breit) 1 H, austauschbar mit $D_2O$, ppm. |

Beispiel 7

$N^1$-[2-[N-(5-Brom-3-methyl-pyridin-2-yl)-benzylamino]ethyl]-$N^2$-[3-(1H-imidazol-4-yl)propyl]guanidin-trihydrochlorid

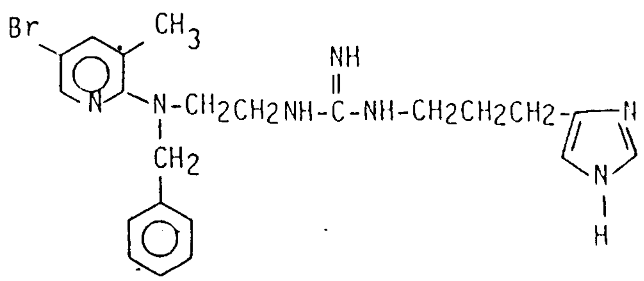

x 3 HCl

1,15 g (2,0 mmol) $N^1$-Benzoyl-$N^2$-[2-[N-(5-brom-3-methylpyridin-2-yl)-benzylamino]ethyl]-$N^3$-[3-(1H-imidazol-4-yl)propyl]-guanidin werden in 20 ml konz. Salzsäure 20 Stunden gekocht. Die wäßrige Lösung wird etwa auf die Hälfte eingeengt und mit 3 x 20 ml Diethylether extrahiert.

Danach wird die wäßrige Phase filtriert, im Vakuum zur Trockene eingedampft und noch zweimal mit jeweils 20 ml absolutem Ethanol im Vakuum eingedampft. Der Rückstand wird aus Isopropanol umkristallisiert.

Ausbeute: 0,82 g (71%) farbloser, sehr hygroskopischer Feststoff.

$C_{22}H_{31}BrCl_3N_7$ (579,80)

| | |
|---|---|
| $^1$H-NMR-Daten: (CD$_3$OD, TMS als interner Standard) | δ = 1,80 - 2,18 (m) 2 H<br>2,61 (s) 3 H,<br>2,89 (t) 2 H,<br>3,34 (t) 2 H,<br>3,60 (m) 2 H,<br>3,83 (m) 2 H,<br>4,15 (t) 2 H,<br>4,9 (breit) 7 H,<br>7,37 - 7,55 (m) 6 H,<br>8,84 (d) 1 H,<br>8,92 (d) 2 H, ppm. |

Beispiel 8

$N^1$-[4-(5-Brom-3-methyl-pyridin-2-yl)butyl]-$N^2$[3-(1H-imidazol-4-yl)propyl]guanidin-trihydrochlorid

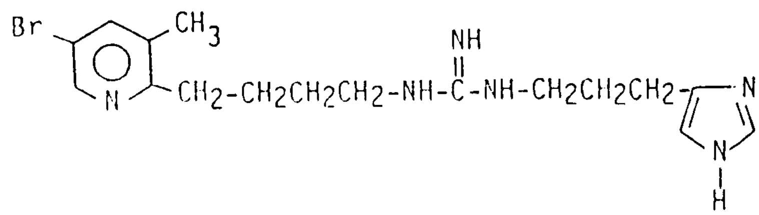 x 3 HCl

1,00 g (2 mmol) $N^1$-Benzoyl-$N^2$[4-(5-brom-3-methyl-pyridin-2-yl)butyl]-$N^3$-[3-(1H-imidazol-4-yl)propyl]-guanidin werden in 20 ml konz. Salzsäure 18 Stunden gekocht. Die nach Abkühlen auf 40 ml verdünnte wäßrige Lösung wird mit 4 x 20 ml Diethylether extrahiert, filtriert und im Vakuum eingedampft. Der Rückstand wird zweimal in 20 ml absolutem Ethanol aufgenommen und eingedampft. Das erhaltene Rohprodukt wird dann mit Natriummethylat in die Base überführt und an Aluminiumoxid mit Essigester/Methanol (1:1) chromatographiert. Die Hauptfraktion wird nach Eindampfen in 5 ml Wasser aufgenommen, mit 0,5 ml konz. Salzsäure versetzt und im Vakuum eingedampft. Nach abermaligem Eindampfen mit 20 ml absolutem Ethanol erhält man 0,62 g (60%) der Titelverbindung in Form eines farblosen, hygroskopischen Feststoffes.
$C_{17}H_{28}BrCl_3N_6$ (502,71)

| | |
|---|---|
| $^1$H-NMR-Daten: (CD$_3$OD, TMS als interner Standard) | $\delta$ = 1,68 - 2,22 (m) 6 H, |
| | 2,61 (s) 3 H, |
| | 2,91 (t) 2 H, |
| | 3,05 - 3,52 (m) 6H |
| | 4,95 (breit) 7 H, |
| | 7,61 (s) 1 H, |
| | 8,89 (d) 1 H, |
| | 9,10 (d) 2 H, ppm. |

## Patentansprüche

**Patentansprüche für folgende Vertragsstaaten : AT, BE, CH, DE, DK, FR, GB, IT, LI, LU, NL, SE**

**1.** Verwendung von Guanidinderivaten der allgemeinen Formel II

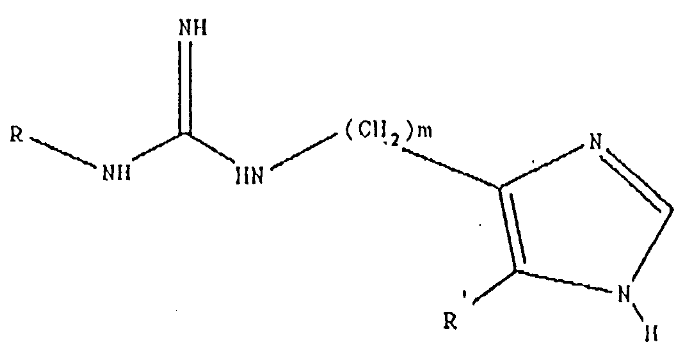

**(II)**

in der R die Gruppierung

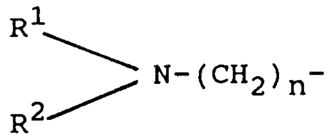

bedeutet, wobei $R^1$ für eine unsubstituierte oder eine ein- oder zweifach mit Halogenatomen, $C_1$-$C_3$-Alkylgruppen oder $C_1$-$C_3$-Alkoxygruppen substituierte Phenylgruppe oder einen unsubstituierten oder einen ein- oder zweifach mit Halogenatomen, $C_1$-$C_3$-Alkylgruppen oder $C_1$-$C_3$-Alkoxygruppen substituierten Pyridinring steht, $R^2$ für ein Wasserstoffatom, eine $C_1$-$C_3$-Alkylgruppe, eine gegebenenfalls ein- oder zweifach mit Halogenatomen, $C_1$-$C_3$-Alkylgruppen oder $C_1$-$C_3$-Alkoxygruppen substituierte Phenylgruppe, eine unsubstituierte oder ein- oder zweifach mit Halogenatomen, $C_1$-$C_3$-Alkylgruppen oder $C_1$-$C_3$-Alkoxygruppen substituierte Benzyloder Heteroarylmethylgruppe steht und n den Wert 2, 3 oder 4 hat,
oder in der R die Gruppierung

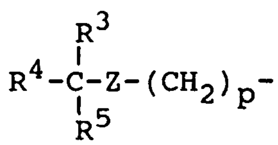

bedeutet, worin $R^3$ für eine unsubstituierte oder ein- oder zweifach mit Halogenatomen, $C_1$-$C_3$-Alkylgruppen oder $C_1$-$C_3$-Alkoxygruppen substituierten Phenylring oder Pyridinring steht, $R^4$ ein Wasserstoffatom oder eine gegebenenfalls ein- oder zweifach mit Halogenatomen, $C_1$-$C_3$-Alkylgruppen oder $C_1$-$C_3$-Alkoxygruppen substituierte Phenylgruppe bedeutet, $R^5$ für ein Wasserstoffatom oder eine Methyl- oder Hydroxygruppe und Z für eine Einfachbindung, ein Sauerstoffatom oder ein Schwefelatom stehen und p den Wert 2 oder 3 hat, m den Wert 2 oder 3 hat und R' ein Wasserstoffatom oder eine Methylgruppe bedeutet, sowie deren physiologisch annehmbaren Salze zur Herstellung eines Arzneimittels mit NPY-antagonistischer Wirkung.

2. Verwendung nach Anspruch 1, dadurch **gekennzeichnet,** daß in der allgemeinen Formel II R für eine der Gruppen
2-(Diphenylmethoxy)ethyl, 2-[Bis-(4-fluorphenyl)-methoxy]ethyl,
2-[Bis-(4-chlorphenyl)methoxy]ethyl,
3-(4-Fluorphenyl)-3-(pyridin-2-yl)propyl,
3-(3,4-Difluorphenyl)-3-(pyridin-2-yl)propyl,
3-(3,5-Difluorphenyl)-3-(pyridin-2-yl)propyl,
3-(4-Chlorphenyl)-3-(pyridin-2-yl)propyl,
3-(3,4-Dichlorphenyl)-3-(pyridin-2-yl)propyl,
3-(4-Fluorphenyl)-3-(pyridin-3-yl)propyl,
2-[N-(5-Brom-3-methyl-pyridin-2-yl)-benzylamino]ethyl,
2-[N-(5-Brom-3-methyl-pyridin-2-yl)-(4-chlorbenzyl)amino]ethyl,
4-(5-Brom-3-methyl-pyridin-2-yl)butyl,
3-(5-Brom-3-methyl-pyridin-2-yl)propyl,
4-(5-Brom-pyridin-2-yl)butyl,
3-(5-Brom-pyridin-2-yl)propyl,
3-(4-Chlorphenyl)-3-phenylpropyl,
3-(4-Fluorphenyl)-3-phenylpropyl,
3,3-Bis-(4-fluorphenyl)propyl oder
3,3-Bis-(4-chlorphenyl)propyl
steht.

3. Verwendung von $N^1$-[3-(1H-Imidazol-4-yl)propyl]-$N^2$-[2-[(pyridin-3-yl)methylthio]ethyl]-guanidin und der physiologisch annehmbaren Salze davon für den Zweck nach Anspruch 1.

4. Verwendung von $N^1$-[3-(1H-Imidazol-4-yl)propyl]-$N^2$-(3,3-diphenylpropyl)guanidin und der physiologisch annehmbaren Salze davon für den Zweck nach Anspruch 1.

5. Verwendung von $N^1$-[3-(1H-Imidazol-4-yl)propyl]-$N^2$-[2-[(pyridin-2-yl)-amino]ethyl]-guanidin und der physiologisch annehmbaren Salze davon für den Zweck nach Anspruch 1.

6. Verwendung von $N^1$-[3-[(5-Brom-3-methyl-pyridin-2-yl)-amino]propyl]-$N^2$-[3-(1H-imidazol-4-yl)propyl]-guanidin und der physiologisch annehmbaren Salze davon für den Zweck nach Anspruch 1.

7. Verwendung von $N^1$-[3-(1H-Imidazol-4-yl)propyl]-$N^2$-[2-(diphenylmethoxy)ethyl]-guanidin und der physiologisch annehmbaren Salze davon für den Zweck nach Anspruch 1.

8. Verwendung von $N^1$-[3-(3,5-Difluorphenyl)-3-(pyridin-2-yl)propyl]-$N^2$-[3-(1H-imidazol-4-yl)propyl]-guanidin und der physiologisch annehmbaren Salze davon für den Zweck nach Anspruch 1.

9. Verwendung von $N^1$-[2-[N-(5-Brom-3-methyl-pyridin-2-yl)-benzylamino]ethyl]-$N^2$-[3-(1H-imidazol-4-yl)-propyl]guanidin und der physiologisch annehmbaren Salze davon für den Zweck nach Anspruch 1.

10. Verwendung von $N^1$-[4-(5-Brom-3-methyl-pyridin-2-yl)butyl]-$N^2$-[3-(1H-imidazol-4-yl)propyl]-guanidin und der physiologisch annehmbaren Salze davon für den Zweck nach Anspruch 1.

**Patentansprüche für folgende Vertragsstaaten : ES, GR**

1. Verfahren zur Herstellung eines Arzneimittels mit NPY-antagonistischer Wirkung, dadurch **gekennzeichnet,** daß man ein Guanidinderivat der allgemeinen Formel II

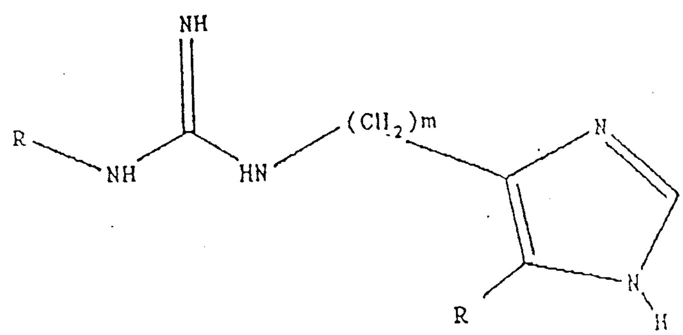

(II)

in der R die Gruppierung

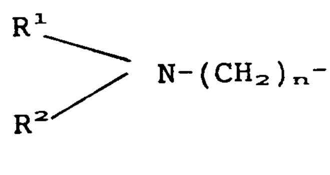

bedeutet, wobei $R^1$ für eine unsubstituierte oder eine ein- oder zweifach mit Halogenatomen, $C_1$-$C_3$-Alkylgruppen oder $C_1$-$C_3$-Alkoxygruppen substituierte Phenylgruppe oder einen unsubstituierten oder einen ein- oder zweifach mit Halogenatomen, $C_1$-$C_3$-Alkylgruppen oder $C_1$-$C_3$-Alkoxygruppen substituierten Pyridinring steht, $R^2$ für ein Wasserstoffatom, eine $C_1$-$C_3$-Alkylgruppe, eine gegebenenfalls ein- oder zweifach mit Halogenatomen, $C_1$-$C_3$-Alkylgruppen oder $C_1$-$C_3$-Alkoxygruppen substituierte Phenyl-

gruppe, eine unsubstituierte oder ein- oder zweifach mit Halogenatomen, $C_1$-$C_3$-Alkylgruppen oder $C_1$-$C_3$-Alkoxygruppen substituierte Benzyl- oder Heteroarylmethylgruppe steht und n den Wert 2, 3 oder 4 hat,
oder in der R die Gruppierung

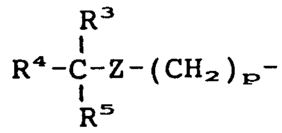

bedeutet, worin $R^3$ für eine unsubstituierte oder ein- oder zweifach mit Halogenatomen, $C_1$-$C_3$-Alkylgruppen oder $C_1$-$C_3$-Alkoxygruppen substituierten Phenylring oder Pyridinring steht, $R^4$ ein Wasserstoffatom oder eine gegebenenfalls ein- oder zweifach mit Halogenatomen, $C_1$-$C_3$-Alkylgruppen oder $C_1$-$C_3$-Alkoxygruppen substituierte Phenylgruppe bedeutet, $R^5$ für ein Wasserstoffatom oder eine Methyl- oder Hydroxygruppe und Z für eine Einfachbindung, ein Sauerstoffatom oder ein Schwefelatom stehen und p den Wert 2 oder 3 hat, m den Wert 2 oder 3 hat und R' ein Wasserstoffatom oder eine Methylgruppe bedeutet, oder ein physiologisch annehmbares Salz davon mit üblichen Hilfs- und Trägerstoffen vermischt.

2. Verfahren nach Anspruch 1, dadurch **gekennzeichnet,** daß in der allgemeinen Formel II R für eine der Gruppen
2-(Diphenylmethoxy)ethyl, 2-[Bis-(4-fluorphenyl)-methoxy]ethyl,
2-[Bis-(4-chlorphenyl)methoxy]ethyl,
3-(4-Fluorphenyl)-3-(pyridin-2-yl)propyl,
3-(3,4-Difluorphenyl)-3-(pyridin-2-yl)propyl,
3-(3,5-Difluorphenyl)-3-(pyridin-2-yl)propyl,
3-(4-Chlorphenyl)-3-(pyridin-2-yl)propyl,
3-(3,4-Dichlorphenyl)-3-(pyridin-2-yl)propyl,
3-(4-Fluorphenyl)-3-(pyridin-3-yl)propyl,
2-[N-(5-Brom-3-methyl-pyridin-2-yl)-benzylamino]ethyl,
2-[N-(5-Brom-3-methyl-pyridin-2-yl)-(4-chlorbenzyl)amino]ethyl,
4-(5-Brom-3-methyl-pyridin-2-yl)butyl,
3-(5-Brom-3-methyl-pyridin-2-yl)propyl,
4-(5-Brom-pyridin-2-yl)butyl,
3-(5-Brom-pyridin-2-yl)propyl,
3-(4-Chlorphenyl)-3-phenylpropyl,
3-(4-Fluorphenyl)-3-phenylpropyl,
3,3-Bis-(4-fluorphenyl)propyl oder
3,3-Bis-(4-chlorphenyl)propyl
steht.

3. Verfahren nach Anspruch 1, dadurch **gekennzeichnet,** daß man als Guanidinderivat $N^1$-(3-(1H-Imidazol-4-yl)propyl]-$N^2$-[2-[(pyridin-3-yl)methylthio]ethyl]-guanidin oder ein physiologisch annehmbares Salz davon verwendet.

4. Verfahren nach Anspruch 1, dadurch **gekennzeichnet,** daß man als Guanidinderivat $N^1$-[3-(1H-Imidazol-4-yl)propyl]-$N^2$-(3,3-diphenylpropyl)guanidin oder ein physiologisch annehmbares Salz davon verwendet.

5. Verfahren nach Anspruch 1, dadurch **gekennzeichnet,** daß man als Guanidinderivat $N^1$-[3-(1H-Imidazol-4-yl)propyl]-$N^2$-[2-[(pyridin-2-yl)-amino]ethyl]-guanidin oder ein physiologisch annehmbares Salz davon verwendet.

6. Verfahren nach Anspruch 1, dadurch **gekennzeichnet,** daß man als Guanidinderivat $N^1$-[3-[(5-Brom-3-methyl-pyridin-2-yl)amino]propyl]-$N^2$-[3-(1H-imidazol-4-yl)propyl]-guanidin oder ein physiologisch annehmbares Salz davon verwendet.

7. Verfahren nach Anspruch 1, dadurch **gekennzeichnet,** daß man als Guanidinderivat $N^1$-[3-(1H-Imidazol-4-yl)propyl]-$N^2$-[2-(diphenylmethoxy)ethyl]-guanidin oder ein physiologisch annehmbares Salz davon verwendet.

8. Verfahren nach Anspruch 1, dadurch **gekennzeichnet,** daß man als Guanidinderivat $N^1$-[3-(3,5-Difluorphenyl)-3-(pyridin-2-yl)propyl]-$N^2$-[3-(1H-imidazol-4-yl)propyl]-guanidin oder ein physiologisch annehmbares Salz davon verwendet.

9. Verfahren nach Anspruch 1, dadurch **gekennzeichnet,** daß man als Guanidinderivat $N^1$-[2-[N-(5-Brom-3-methyl-pyridin-2-yl)-benzylamino]ethyl]-$N^2$-[3-(1H-imidazol-4-yl)propyl]guanidin oder ein physiologisch annehmbares Salz davon verwendet.

10. Verfahren nach Anspruch 1, dadurch **gekennzeichnet,** daß man als Guanidinderivat $N^1$-[4-(5-Brom-3-methyl-pyridin-2-yl)butyl]-$N^2$-[3-(1H-imidazol-4-yl)propyl]-guanidin oder ein physiologisch annehmbares Salz davon verwendet.

**Claims**

**Claims for the following Contracting States : AT, BE, CH, DE, DK, FR, GB, IT, LI, LU, NL, SE**

1. The use of guanidine derivatives corresponding to general formula II

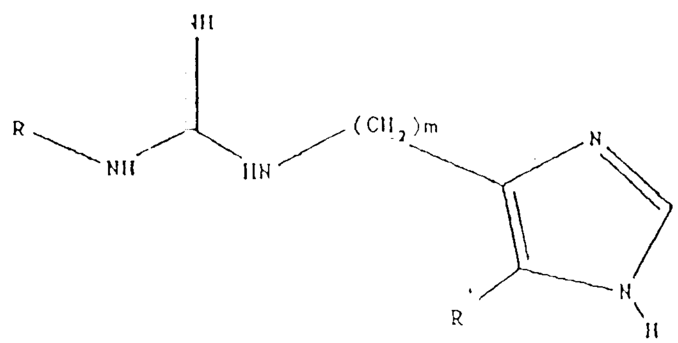

(II)

in which R represents the group:

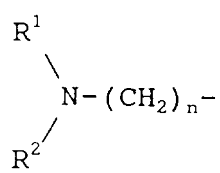

where $R^1$ is an unsubstituted phenyl group or a phenyl group substituted once or twice by halogen atoms, $C_{1-3}$ alkyl groups or $C_{1-3}$ alkoxy groups or an unsubstituted pyridine ring or a pyridine ring substituted once or twice by halogen atoms, $C_{1-3}$ alkyl groups or $C_{1-3}$ alkoxy groups, $R^2$ is a hydrogen atom, a $C_{1-3}$ alkyl group, a phenyl group optionally substituted once or twice by halogen atoms, $C_{1-3}$ alkyl groups or $C_{1-3}$ alkoxy groups, an unsubstituted benzyl or heteroarylmethyl group or a benzyl or heteroarylmethyl group substituted once or twice by halogen atoms, $C_{1-3}$ alkyl groups or $C_{1-3}$ alkoxy groups and n has a value of 2, 3 or 4,
or in which R represents the group:

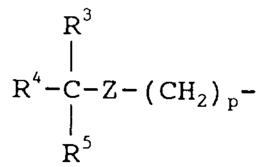

where $R^3$ is an unsubstituted phenyl ring or pyridine ring or a phenyl ring or pyridine ring substituted once or twice by halogen atoms, $C_{1-3}$ alkyl groups or $C_{1-3}$ alkoxy groups, $R^4$ is a hydrogen atom or a phenyl group optionally substituted once or twice by halogen atoms, $C_{1-3}$ alkyl groups or $C_{1-3}$ alkoxy groups, $R^5$ is a hydrogen atom or a methyl group and Z is a single bond, an oxygen atom or a sulfur atom and p has a value of 2 or 3, m has a value of 2 or 3 and R' is a hydrogen atom or a methyl group,
and physiologically acceptable salts thereof for the production of a medicament with an NPY-antagonistic effect.

2. The use claimed in claim 1, characterized in that, in general formula II, R represents one of the following groups:
2-(diphenylmethoxy)-ethyl,
2-[bis-(4-fluorophenyl)-methoxy]-ethyl,
2-[bis-(4-chlorophenyl)-methoxy]-ethyl,
3-(4-fluorophenyl)-3-(pyridin-2-yl)-propyl,
3-(3,4-difluorophenyl)-3-(pyridin-2-yl)-propyl,
3-(3,5-difluorophenyl)-3-(pyridin-2-yl)-propyl,
3-(4-chlorophenyl)-3-(pyridin-2-yl)-propyl,
3-(3,4-dichlorophenyl)-3-(pyridin-2-yl)-propyl,
3-(4-fluorophenyl)-3-(pyridin-3-yl)-propyl,
2-[N-(5-bromo-3-methylpyridin-2-yl)-benzylamino]-ethyl,
2-[N-(5-bromo-3-methylpyridin-2-yl)-(4-chlorobenzyl)-amino]-ethyl,
4-(5-bromo-3-methylpyridin-2-yl)-butyl,
3-(5-bromo-3-methylpyridin-2-yl)-propyl,
4-(5-bromopyridin-2-yl)-butyl,
3-(5-bromopyridin-2-yl)-propyl,
3-(4-chlorophenyl)-3-phenylpropyl,
3-(4-fluorophenyl)-3-phenylpropyl,
3,3-bis-(4-fluorophenyl)-propyl or
3,3-bis-(4-chlorophenyl)-propyl.

3. The use of $N^1$-[3-(1H-imidazol-4-yl)-propyl]-$N^2$-[2-[(pyridin-3-yl)-methylthio]ethyl]-guanidine and physiologically acceptable salts thereof for the purpose according to claim 1.

4. The use of $N^1$-[3-(1H-imidazol-4-yl)-propyl]-$N^2$-(3,3-diphenylpropyl)-guanidine and physiologically acceptable salts thereof for the purpose according to the claim 1.

5. The use of $N^1$-[3-(1H-imidazol-4-yl)-propyl]-$N^2$-[2-[(pyridin-2-yl)-amino]ethyl]-guanidine and physiologically acceptable salts thereof for the purpose according to claim 1.

6. The use of $N^1$-[3-[(5-bromo-3-methylpyridin-2-yl)-amino]propyl]-$N^2$-[3-(1H-imidazol-4-yl)-propyl]-guanidine and physiologically acceptable salts thereof for the purpose according to claim 1.

7. The use of $N^1$-[3-(1H-imidazol-4-yl)-propyl]-$N^2$-[2-(diphenylmethoxy)-ethyl]-guanidine and physiologically acceptable salts thereof for the purpose according to claim 1.

8. The use of $N^1$-[3-(3,5,-difluorophenyl)-3-(pyridin-2-yl)-propyl]-$N^2$-[3-(1H-imidazol-4-yl)-propyl]-guanidine and physiologically acceptable salts thereof for the purpose according to claim 1.

9. The use of $N^1$-[2-[N-(5-bromo-3-methylpyridin-2-yl)-benzylamino]ethyl]-$N^2$-[3-(1H-imidazol-4-yl)-propyl]-guanidine and physiologically acceptable salts thereof for the purpose according to claim 1.

**10.** The use of $N^1$-[4-(5-bromo-3-methylpyridin-2-yl)butyl]-$N^2$-[3-(1H-imidazol-4-yl)-propyl]-guanidine and physiologically acceptable salts thereof for the purpose according to claim 1.

**Claims for the following Contracting States : ES, GR**

**1.** A process for the production of a medicament with an NPY-antagonistic effect, characterized in that a guanidine derivative corresponding to general formula II:

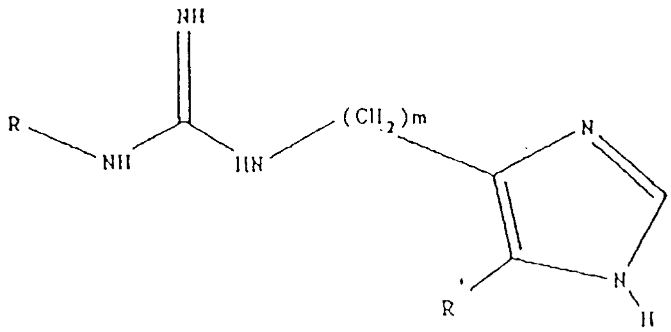

(II)

in which R represents the group:

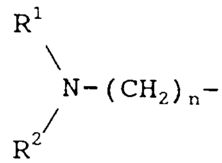

where $R^1$ is an unsubstituted phenyl group or a phenyl group substituted once or twice by halogen atoms, $C_{1-3}$ alkyl groups or $C_{1-3}$ alkoxy groups or an unsubstituted pyridine ring or a pyridine ring substituted once or twice by halogen atoms, $C_{1-3}$ alkyl groups or $C_{1-3}$ alkoxy groups, $R^2$ is a hydrogen atom, a $C_{1-3}$ alkyl group, a phenyl group optionally substituted once or twice by halogen atoms, $C_{1-3}$ alkyl groups or $C_{1-3}$ alkoxy groups, an unsubstituted benzyl or heteroarylmethyl group or a benzyl or heteroarylmethyl group substituted once or twice by halogen atoms, $C_{1-3}$ alkyl groups or $C_{1-3}$ alkoxy groups and n has a value of 2, 3 or 4,
or in which R represents the group:

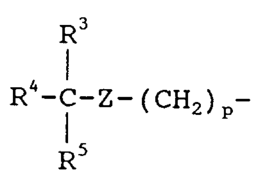

where $R^3$ is an unsubstituted phenyl ring or pyridine ring or a phenyl ring or pyridine ring substituted once or twice by halogen atoms, $C_{1-3}$ alkyl groups or $C_{1-3}$ alkoxy groups, $R^4$ is a hydrogen atom or a phenyl group optionally substituted once or twice by halogen atoms, $C_{1-3}$ alkyl groups or $C_{1-3}$ alkoxy groups, $R^5$ is a hydrogen atom or a methyl group and Z is a single bond, an oxygen atom or a sulfur atom and p has a value of 2 or 3, m has a value of 2 or 3 and R' is a hydrogen atom or a methyl group,
or a physiological acceptable salt thereof is mixed with typical auxiliaries and supports.

**2.** A process as claimed in claim 1, characterized in that, in general formula II, R represents one of the following groups:

2-(diphenylmethoxy)-ethyl,
2-[bis-(4-fluorophenyl)-methoxy]-ethyl,
2-[bis-(4-chlorophenyl)-methoxy]-ethyl,
3-(4-fluorophenyl)-3-(pyridin-2-yl)-propyl,
3-(3,4-difluorophenyl)-3-(pyridin-2-yl)-propyl,
3-(3,5-difluorophenyl)-3-(pyridin-2-yl)-propyl,
3-(4-chlorophenyl)-3-(pyridin-2-yl)-propyl,
3-(3,4-dichlorophenyl)-3-(pyridin-2-yl)-propyl,
3-(4-fluorophenyl)-3-(pyridin-3-yl)-propyl,
2-[N-(5-bromo-3-methylpyridin-2-yl)-benzylamino]-ethyl,
2-[N-(5-bromo-3-methylpyridin-2-yl)-(4-chlorobenzyl)-amino]-ethyl,
4-(5-bromo-3-methylpyridin-2-yl)-butyl,
3-(5-bromo-3-methylpyridin-2-yl)-propyl,
4-(5-bromopyridin-2-yl)-butyl,
3-(5-bromopyridin-2-yl)-propyl,
3-(4-chlorophenyl)-3-phenylpropyl,
3-(4-fluorophenyl)-3-phenylpropyl,
3,3-bis-(4-fluorophenyl)-propyl or
3,3-bis-(4-chlorophenyl)-propyl.

**3.** A process as claimed in claim 1, characterized in that $N^1$-[3-(1H-imidazol-4-yl)-propyl]-$N^2$-[2-[(pyridin-3-yl)-methylthio]ethyl]-guanidine or a physiologically acceptable salt thereof is used as the guanidine derivative.

**4.** A process as claimed in claim 1, characterized in that $N^1$-[3-(1H-imidazol-4-yl)-propyl]-$N^2$-(3,3-diphenylpropyl)-guanidine or a physiologically acceptable salt thereof is used as the guanidine derivative.

**5.** A process as claimed in claim 1, characterized in that $N^1$-[3-(1H-imidazol-4-yl)-propyl]-$N^2$-[2-[(pyridin-2-yl)-amino]ethyl]-guanidine or a physiologically acceptable salt thereof is used as the guanidine derivative.

**6.** A process as claimed in claim 1, characterized in that$N^1$-[3-[(5-bromo-3-methylpyridin-2-yl)-amino]-propyl]-$N^2$-[3-(1H-imidazol-4-yl)-propyl]-guanidine or a physiologically acceptable salt thereof is used as the guanidine derivative.

**7.** A process as claimed in claim 1, characterized in that $N^1$-[3-(1H-imidazol-4-yl)-propyl]-$N^2$-[2-(diphenylmethoxy)-ethyl]-guanidine or a physiologically acceptable salt thereof is used as the guanidine derivative.

**8.** A process as claimed in claim 1, characterized in that $N^1$-[3-(3,5,-difluorophenyl)-3-(pyridin-2-yl)-propyl]-$N^2$-[3-(1H-imidazol-4-yl)-propyl]-guanidine or a physiologically acceptable salt thereof is used as the guanidine derivative.

**9.** A process as claimed in claim 1, characterized in that $N^1$-[2-[N-(5-bromo-3-methylpyridin-2-yl)-benzylamino]-ethyl]-$N^2$-[3-(1H-imidazol-4-yl)-propyl]-guanidine or a physiologically acceptable salt thereof is used as the guanidine derivative.

**10.** A process as claimed in claim 1, characterized in that $N^1$-[4-(5-bromo-3-methylpyridin-2-yl)-butyl]-$N^2$-[3-(1H-imidazol-4-yl)-propyl]-guanidine or a physiologically acceptable salt thereof is used as the guanidine derivative.

## Revendications

**Revendications pour les Etats contractants suivants : AT, BE, CH, DE, DK, FR, GB, IT, LI, LU, NL, SE**

**1.** Utilisation de dérivés de guanidine de formule générale II :

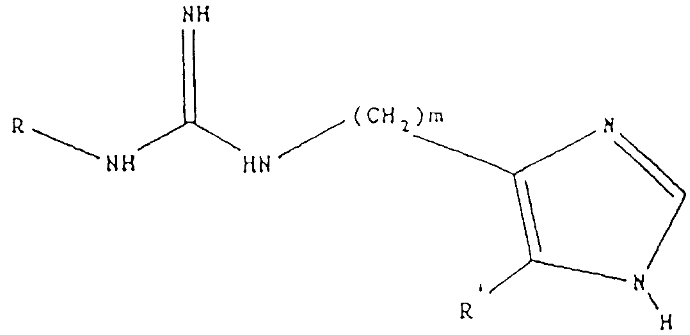

(II)

dans laquelle R signifie le groupement :

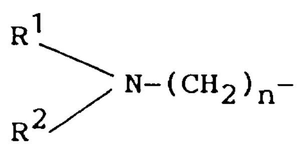

où $R^1$ représente un groupe phényle non substitué ou un groupe phényle mono- ou disubstitué par des atomes d'halogène, par des groupes alkyles en $C_1$-$C_3$ ou par des groupes alcoxy en $C_1$-$C_3$, ou un cycle pyridique non substitué ou mono- ou disubstitué par des atomes d'halogène, par des groupes alkyles en $C_1$-$C_3$ ou par des groupes alcoxy en $C_1$-$C_3$, $R^2$ représente un atome d'hydrogène, un groupe alkyle en $C_1$-$C_3$, un groupe phényle éventuellement mono- ou disubstitué par des atomes d'halogène, par des groupes alkyles en $C_1$-$C_3$ ou par des groupes alcoxy en $C_1$-$C_3$, un groupe benzyle ou hétéroarylméthyle non substitue ou mono- ou disubstitué par des atomes d'halogène, par des groupes alkyles en $C_1$-$C_3$ ou par des groupes alcoxy en $C_1$-$C_3$, et n vaut 2, 3 ou 4, ou dans laquelle R signifie le groupement :

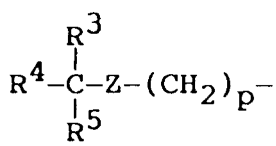

où $R^3$ représente un cycle phénylique ou pyridique non substitué ou mono- ou disubstitué par des atomes d'halogène, par des groupes alkyles en $C_1$-$C_3$ ou par des groupes alcoxy en $C_1$-$C_3$, $R^4$ représente un atome d'hydrogène ou un groupe phényle éventuellement mono- ou disubstitué par des atomes d'halogène, par des groupes alkyles en $C_1$-$C_3$ ou par des groupes alcoxy en $C_1$-$C_3$, $R^5$ représente un atome d'hydrogène ou un groupe méthyle ou hydroxy, Z représente une liaison simple, un atome d'oxygène ou un atome de soufre, et p vaut 2 ou 3, m vaut 2 ou 3, et R' signifie un atome d'hydrogène ou un groupe méthyle,
ainsi que leurs sels physiologiquement acceptables, pour la préparation d'un médicament ayant une activité antagoniste du neuropeptide Y.

**2.** Utilisation selon la revendication 1, caractérisée en ce que, dans la formule générale II, R représente un des groupes 2-(diphénylméthoxy)éthyle, 2-[bis-(4-fluorophényl)méthoxy]éthyle, 2-[bis(4-chlorophényl)-méthoxy]éthyle, 3-(4-fluorophényl)-3-(pyridine-2-yl)propyle, 3-(3,4-difluorophényl)-3-(pyridine-2-yl)-propyle, 3-(3,5-difluorophényl)-3-(pyridine-2-yl)propyle, 3-(4-chlorophényl)-3-(pyridine-2-yl)propyle, 3-(3,4-dichlorophényl)-3-(pyridine-2-yl)propyle, 3-(4-fluorophényl)-3-(pyridine-3-yl)propyle, 2-[N-(5-bromo-3-méthyl-pyridine-2-yl)benzylamino]éthyle, 2-[N-(5-bromo-3-méthyl-pyridine-2-yl)-(4-chlorobenzyl)-amino]-éthyle, 4-(5-bromo-3-méthyl-pyridine-2-yl)butyle, 3-(5-bromo-3-méthyl-pyridine-2-yl)propyle, 4-(5-bromo-pyridine-2-yl)butyle, 3-(5-bromo-pyridine-2-yl)propyle, 3-(4-chlorophényl)-3-phénylpropyle, 3-(4-fluorophényl)-3-phénylpropyle, 3,3-bis(4-fluorophényl)propyle ou 3,3-bis(4-chlorophényl)propyle.

**3.** Utilisation de $N^1$-[3-(1H-imidazol-4-yl)propyl]-$N^2$-[2-[(pyridine-3-yl)méthylthio]éthyl]guanidine et de ses sels physiologiquement acceptables pour l'objectif selon la revendication 1.

**4.** Utilisation de $N^1$-[3-(1H-imidazol-4-yl)propyl]-$N^2$-(3,3-diphénylpropyl)guanidine et de ses sels physiologiquement acceptables pour l'objectif selon la revendication 1.

**5.** Utilisation de $N^1$-[3-(1H-imidazol-4-yl)propyl]-$N^2$-[2-[(pyridine-2-yl)amino]éthyl]guanidine et de ses sels physiologiquement acceptables pour l'objectif selon la revendication 1.

**6.** Utilisation de $N^1$-[3-[(5-bromo-3-méthyl-pyridine-2-yl)amino]propyl]-$N^2$-[3-(1H-imidazol-4-yl)propyl]-guanidine et de ses sels physiologiquement acceptables pour l'objectif selon la revendication 1.

**7.** Utilisation de $N^1$-[3-(1H-imidazol-4-yl)propyl]-$N^2$-[2-(diphénylméthoxy)éthyl]guanidine et de ses sels physiologiquement acceptables pour l'objectif selon la revendication 1.

**8.** Utilisation de $N^1$-[3-(3,5-difluorophényl)-3-(pyridine-2-yl)propyl]-$N^2$-[3-(1H-imidazol-4-yl)propyl]guanidine et de ses sels physiologiquement acceptables pour l'objectif selon la revendication 1.

**9.** Utilisation de $N^1$-[2-[N-(5-bromo-3-méthyl-pyridine-2-yl)benzylamino]éthyl]-$N^2$-[3-(1H-imidazol-4-yl)-propyl]guanidine et de ses sels physiologiquement acceptables pour l'objectif selon la revendication 1.

**10.** Utilisation de $N^1$-[4-(5-bromo-3-méthyl-pyridine-2-yl)butyl]-$N^2$-[3-(1H-imidazol-4-yl)propyl]guanidine et de ses sels physiologiquement acceptables pour l'objectif selon la revendication 1.

**Revendications pour les Etats contractants suivants : ES, GR**

**1.** Procédé de préparation d' un médicament ayant une activité antagoniste du neuropeptide Y, caractérisé en ce qu'on mélange un dérivé de guanidine de formule générale II :

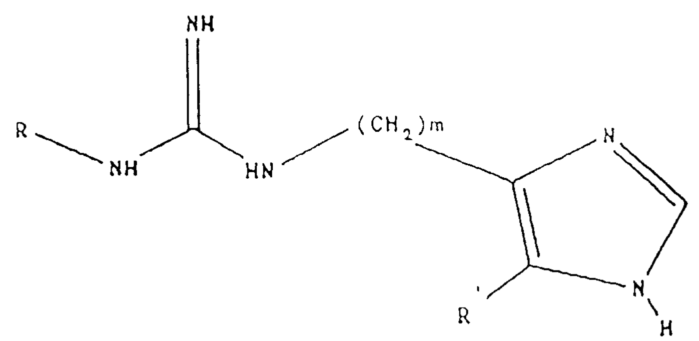

(II)

dans laquelle R signifie le groupement :

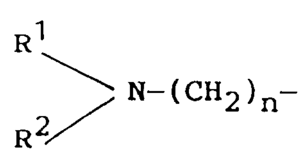

où $R^1$ représente un groupe phényle non substitué ou un groupe phényle mono- ou disubstitué par des atomes d'halogène, par des groupes alkyles en $C_1$-$C_3$ ou par des groupes alcoxy en $C_1$-$C_3$, ou un cycle pyridique non substitué ou mono- ou disubstitué par des atomes d'halogène, par des groupes alkyles en $C_1$-$C_3$ ou par des groupes alcoxy en $C_1$-$C_3$, $R^2$ représente un atome d'hydrogène, un groupe alkyle en $C_1$-$C_3$, un groupe phényle éventuellement mono- ou disubstitué par des atomes

d'halogène, par des groupes alkyles en $C_1$-$C_3$ ou par des groupes alcoxy en $C_1$-$C_3$, un groupe benzyle ou hétéroarylméthyle non substitue ou mono- ou disubstitué par des atomes d'halogène, par des groupes alkyles en $C_1$-$C_3$ ou par des groupes alcoxy en $C_1$-$C_3$, et n vaut 2, 3 ou 4, ou dans laquelle R signifie le groupement :

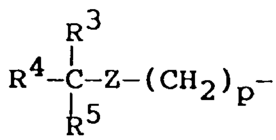

où $R^3$ représente un cycle phénylique ou pyridique non substitué ou mono- ou disubstitué par des atomes d'halogène, par des groupes alkyles en $C_1$-$C_3$ ou par des groupes alcoxy en $C_1$-$C_3$, $R^4$ représente un atome d'hydrogène ou un groupe phényle éventuellement mono- ou disubstitué par des atomes d'halogène, par des groupes alkyles en $C_1$-$C_3$ ou par des groupes alcoxy en $C_1$-$C_3$, $R^5$ représente un atome d'hydrogène ou un groupe méthyle ou hydroxy, Z représente une liaison simple, un atome d'oxygène ou un atome de soufre, et p vaut 2 ou 3, m vaut 2 ou 3, et R' signifie un atome d'hydrogène ou un groupe méthyle,
ou de ses sels physiologiquement acceptables, avec des adjuvants et des supports usuels.

2. Procédé selon la revendication 1, caractérisé en ce que, dans la formule générale II, R représente un des groupes 2-(diphénylméthoxy)éthyle, 2-[bis-(4-fluorophényl)méthoxy]éthyle, 2-[bis(4-chlorophényl)-méthoxy]éthyle, 3-(4-fluorophényl)-3-(pyridine-2-yl)propyle, 3-(3,4-difluorophényl)-3-(pyridine-2-yl)-propyle, 3-(3,5-difluorophényl)-3-(pyridine-2-yl)propyle, 3-(4-chlorophényl)-3-(pyridine-2-yl)propyle, 3-(3,4-dichlorophényl)-3-(pyridine-2-yl)propyle, 3-(4-fluorophényl)-3-(pyridine-3-yl)propyle, 2-[N-(5-bromo-3-méthyl-pyridine-2-yl)benzylamino]éthyle, 2-[N-(5-bromo-3-méthyl-pyridine-2-yl)-(4-chlorobenzyl)-amino]éthyle, 4-(5-bromo-3-méthyl-pyridine-2-yl)butyle, 3-(5-bromo-3-méthyl-pyridine-2-yl)propyle, 4-(5-bromo-pyridine-2-yl)butyle, 3-(5-bromo-pyridine-2-yl)propyle, 3-(4-chlorophényl)-3-phénylpropyle, 3-(4-fluorophényl)-3-phénylpropyle, 3,3-bis(4-fluorophényl)propyleou 3,3-bis(4-chlorophényl)propyle.

3. Procédé selon la revendication 1, caractérisé en ce qu'on utilise, comme dérivé de la guanidine, la $N^1$-[3-(1H-imidazol-4-yl)propyl]-$N^2$-[2-[(pyridine-3-yl)méthylthio]éthyl]guanidineou un de ses sels physiologiquement acceptables.

4. Procédé selon la revendication 1, caractérisé en ce qu'on utilise, comme dérivé de la guanidine, la $N^1$-[3-(1H-imidazol-4-yl)propyl]-$N^2$-(3,3-diphénylpropyl)guanidine ou un de ses sels physiologiquement acceptables.

5. Procédé selon la revendication 1, caractérisé en ce qu'on utilise, comme dérivé de la guanidine, la $N^1$-[3-(1H-imidazol-4-yl)propyl]-$N^2$-[2-[(pyridine-2-yl)amino]éthyl]guanidine ou un de ses sels physiologiquement acceptables.

6. Procédé selon la revendication 1, caractérisé en ce qu'on utilise, comme dérivé de la guanidine, la $N^1$-[3-[(5-bromo-3-méthyl-pyridine-2-yl)amino]propyl]-$N^2$-[3-(1H-imidazol-4-yl)propyl]guanidine ou un de ses sels physiologiquement acceptables.

7. Procédé selon la revendication 1, caractérisé en ce qu'on utilise, comme dérivé de la guanidine, la $N^1$-[3-(1H-imidazol-4-yl)propyl]-$N^2$-[2-(diphénylméthoxy)éthyl]guanidine ou un de ses sels physiologiquement acceptables.

8. Procédé selon la revendication 1, caractérisé en ce qu'on utilise, comme dérivé de la guanidine, la $N^1$-[3-(3,5-difluorophényl)-3-(pyridine-2-yl)propyl]-$N^2$-[3-(1H-imidazol-4-yl)propyl]guanidine ou un de ses sels physiologiquement acceptables.

9. Procédé selon la revendication 1, caractérisé en ce qu'on utilise, comme dérivé de la guanidine, la $N^1$-[2-[N-(5-bromo-3-méthyl-pyridine-2-yl)benzylamino]éthyl]-$N^2$-[3-(1H-imidazol-4-yl)propyl]guanidine ou un de ses sels physiologiquement acceptables.

**10.** Procédé selon la revendication 1, caractérisé en ce qu'on utilise, comme dérivé de la guanidine, la $N^1$-[4-(5-bromo-3-méthyl-pyridine-2-yl)butyl]-$N^2$-[3-(1H-imidazol-4-yl)propyl]guanidine ou un de ses sels physiologiquement acceptables.